# EUROPEAN PATENT SPECIFICATION

(11) **EP 2 080 789 B1**
(45) Date of publication and mention of the grant of the patent: **30.03.2011**
(21) Application number: 08021273.1
(22) Date of filing: 08.12.2008
(51) Int. Cl.: C09C 1/00, C09C 1/40

(54) **Filler pigments**
Füllstoffpigmente
Pigments de charge

(30) Priority: 17.12.2007 EP 07024443
(43) Date of publication of application: 22.07.2009
(73) Proprietor: Merck Patent GmbH, 64293 Darmstadt (DE)
(72) Inventor: Schmidt, Christoph, Dr., 65830 Kriftel (DE); Schoen, Sabine, Dr., 45701 Herten (DE); Noguchi, Tamio, Dr., Iwaki-shi Fukushima ken 972-8322 (JP)

(56) References cited:
- EP-A- 0 142 695
- EP-A- 0 889 099

## Description

This invention relates to filler pigments based on platelet shaped substrates which are coated with a layer containing barium sulfate and at least two metal oxides and/or metal hydroxides. These filler pigments are highly suitable in cosmetic applications.

It is known to use flaky powders, such as, for example, mica, e.g. muscovite or sericite, or clay materials, such as kaolin or talc, as these are starting materials for the preparation of filler powders which are especially useful in cosmetics. These filler powders by themselves do not possess the necessary properties, i.e., adhesion and extension, for the use in diverse kinds of cosmetics like face powders, make-ups, and the like. Therefore, they are conventionally mixed with additives, like titanium dioxide, metal soaps and/or calcium carbonate.

EP 0 142 695 B1 discloses pigments based on mica coated with barium sulfate. These pigments are suitable for the use in foundations like face powders, etc., due to their tactile and optical properties. However, these filler pigments have particular disadvantages like photoactivity and whitish appearance.

The object of the present invention was to provide a photostable filler pigment based on platelet-shaped substrates which shows no gloss, provides good skin-feeling and natural look.

Surprisingly, filler pigments with less or no photoactivity have now been found, which, besides an extraordinarily soft skin feeling, have a good extensibility in applications and show a hiding characteristic which supports natural appearance of the skin. This invention therefore relates to filler pigments, which are characterized in that a platelet-shaped substrate is coated with barium sulfate and at least two metal oxides and/or metal hydroxides.

The invention also relates to a process for the preparation of a flaky filler pigment. Owing to the advantageous properties, the filler pigments according to the invention are universally suitable for a large number of very different applications. The present invention accordingly also relates to the use of these filler pigments in cosmetics, paints, coatings, plastics, films, free-flowing preparations and dry preparations like granules, pellets, etc.

The invention likewise relates to cosmetic formulations, such as, for example, make-ups, compact-powders, loose powders, lipsticks, etc. which comprise the filler pigment according to the invention. The filler pigment is highly suitable as light-diffusing pigment which means it has a balanced relation of transparency, scattering and reflection. Since it scatters light to minimize the visibility of wrinkles, the filler pigment is highly useful for make-ups, anti-wrinkle products and skin correctors.

The filler pigments according to the invention are based on flake-form substrates. Suitable substrates are flakes of natural or synthetic mica, phyllosilicates, silicon dioxide, tin dioxide, zirconium dioxide, glass, aluminium oxide, titanium dioxide, magnesium fluoride and/or iron oxide or mixtures thereof. The substrate of the filler pigment according to the invention preferably consists of mica (synthetic and natural) flakes, Al₂O₃ flakes, glass flakes, talc, kaolin, SiO₂ flakes, and most preferably of mica.

Preferred SiO₂ flakes have a uniform layer thickness and are preferably produced in accordance with the International patent application WO 93/08237 on a continuous belt by solidification and hydrolysis of a water-glass solution. "Uniform layer thickness" here is taken to mean a layer thickness tolerance of from 3 to 10%, preferably from 3 to 6 %, of the total dry layer thickness of the particles. The flake-form silicon dioxide particles are generally in amorphous form.

Preferred base substrates are glass flakes owing to their smooth surfaces and high transparence. The size of the base substrates is not crucial per se and can be matched to the particular application. Particular preference is given to glass flakes having an average thickness of < 2 µm. Thicker flakes generally cannot be employed in common printing processes and in demanding paint finishes. Thin glass flakes provide a better skin feeling compared to thick flakes having thicknesses > 1 µm. Therefore, the glass flakes preferably have thicknesses of < 1 µm, in particular of < 0.9 µm, very particularly preferably of < 0.7 µm. Particular preference is given to glass flakes having thicknesses of 0.25-0.7 µm. The diameter of the glass flakes is preferably 20-200 µm, particularly preferably 10-60 µm, and most preferably ≤ 40 µm. Glass flakes having these dimensions are commercially available and/or can be produced by known processes, such as, for example, tube blowing (Nippon Sheet Glass), spinning process (Glassflake Ltd.)

Particular preference is given to doped or undoped Al₂O₃ flakes. Suitable Al₂O₃ flakes are those for example which are disclosed in JP 3242561 B.

The diameter of the substrates is usually below 100 µm, preferably below 50 and most preferably ≤ 40 µm. The thickness is from 50-2000 nm, preferably 50-1000 nm and particularly preferably 50-500 nm.

The average aspect ratio of the flake-form substrates, i.e. the ratio of the average length measurement value, which corresponds to the average diameter here, to the average thickness measurement value, is usually from 2 to 2000, preferably from 2 to 1000 and particularly preferably from 2 to 200.

Before application of the barium sulfate layer and/or the metal oxide layers on the substrate, a thin dielectric layer where 1.4 < n < 2.7 (n = refractive index), can optionally also be deposited. A coating of this type, for example on glass flakes, can consist, for example of a SiO₂ layer or ZnO layer, preferably of a SiO₂ layer with a thickness of 2-20 nm.

The filler pigments according to the invention contain 5-200 wt.-%, preferably 5-100 wt.-% and most preferably 10-50 wt.-% of barium sulfate based on the substrate.

The barium sulfate layer is combined with at least two metal oxides. The weight ratio of the barium sulfate and the metal oxides may vary from 1 : 10 to 5 : 1 depending on the desired properties, e.g. the hiding power and color of the filler powder.

In the case that the barium sulfate layer contains titanium dioxide the titanium dioxide can be in the anatase or rutile modification. Rutile is the preferred modification. The rutile layer can be prepared according to the the process described in EP 0 271 767.

Preferred metal oxides/hydroxides are selected from TiO₂, SnO₂, ZnO, Fe₂O₃, Fe₃O₄, SiO₂, Al₂O₃, ZrO₂ and the corresponding hydroxides as well as combinations thereof.

In addition the barium sulfate layer may also be doped with carbon black and/or organic or inorganic colorants, where the proportion of doping should not exceed 10 % by weight, based on the BaSO₄ layer.

In a preferred embodiment, additionally the filler pigment can be coated with a colorant selected from the group of Carmine red, Prussian blue, indanthrene brilliant rosa, 1,4-diketo-pyrrolopyrrol derivatives, derivatives of thioindigo-, indigo-, triphenylmethane-, azo-, anthrachinone-, phthalocyanine- or indanthrene, Fe₂O₃, Cr₂O₃, BiVO₄, CoAl₂O₄ or Fe₃O₄.

If the TiO₂ layer essentially consists of rutile, full-area or partial coating with SnO₂ nuclei is preferably carried out before the coating with TiO₂. This very thin SnO₂ layer has maximum thicknesses of 20 nm, preferably ≤ 10 nm, most preferable ≤ 5 nm. The SnO₂ layer may also be distributed on the surface or the substrate as simple dots.

The photostability of the filler pigments can be increased with a SiO₂ layer on the surface of the final filler pigment. These pigments have the further advantage that they do not show any or less reactions with dihydroxy acetone (DHA) which is often used in self-tanning cosmetics.

The base substrate can be coated with a mixed layer containing the barium sulfate and at least two metal oxides. Furthermore, it is possible to apply the metal oxide layer(s) on the base substrate first and secondly the barium sulfate layer or the barium sulfate layer on the base substrate and secondly the metal oxide layer(s). The metal oxide layer consists of at least one metal oxide. The metal oxide layer can be a mixed metal oxide layer consisting of at least two different metal oxides or of two layers of different metal oxides. In general, any order of the oxide layers and the barium sulfate is possible.

Preferred filler pigments have the following layer structures:

Metal oxide 1 and metal oxide 2 can be the same or different. In a preferred embodiment metal oxide 1 and metal oxide 2 are different.

| | | |
|---|---|---|
| substrate | | |
| 1^{st} layer: | BaSO₄ | |
| 2^{nd} layer: | metal oxide 1 | |
| 3^{rd} layer: | metal oxide 2 | or |
| | | |
| substrate | | |
| 1^{st} layer: | metal oxide 1 | |
| 2^{nd} layer: | BaSO₄ | |
| 3^{rd} layer: | metal oxide 2 | or |
| | | |
| substrate | | |
| 1^{st} layer: | metal oxide 1 | |
| 2^{nd} layer: | metal oxide 2 | |
| 3^{rd} layer:, | BaSO₄ | or |
| | | |
| substrate | | |
| 1^{st} layer: | mixture of BaSO₄ + metal oxide 1 | |
| 2^{nd} layer : | metal oxide 2 | or |
| | | |
| substrate | | |
| 1^{st} layer: | metal oxide 1 | |
| 2^{nd} layer: | mixture of BaSO₄ + metal oxide 2 | or |
| substrate | | |
| 1^{st} layer: | mixture of BaSO₄, metal oxide 1 and metal oxide 2. | |

Especially preferred filler pigments have the following layer structures:

substrate + SnO₂ + TiO₂ + BaSO₄

substrate + mixture of SnO₂ + TiO₂ + BaSO₄

substrate + mixture of SnO₂ + TiO₂ + BaSO₄ + SiO₂

substrate + SiO₂ + TiO₂ + BaSO₄

substrate + SiO₂ + SnO₂ + BaSO₄

substrate + SnO₂ + TiO₂ + BaSO₄ + Carmine red

substrate + SnO₂ + TiO₂ + BaSO₄ + Prussian Blue

substrate + SnO₂ + BaSO₄ / TiO₂

substrate + SnO₂ + TiO₂ + BaSO₄ + SiO₂

substrate + BaSO₄ + SnO₂ + TiO₂ + SiO₂

substrate + Al₂O₃ + BaSO₄ + SnO₂ + SiO₂

substrate + ZnO + Al₂O₃ + BaSO₄ + SiO₂

substrate + Al₂O₃ + BaSO₄ + SnO₂

substrate + ZnO + Al₂O₃ + BaSO₄

The filler pigments are prepared in the way that the substrate particles or mixtures of substrate particles are suspended in water, and a hydrolysable barium salt respectively, at least two different metal salt solutions or a metal salt and a silicate solution, preferably a sodium silicate solution, are added at a pH which is suitable for hydrolysis and which is selected in such a way that the barium sulfate and the metal oxides or metal oxide hydroxides are precipitated directly onto the substrates without secondary precipitations occurring. The pH is usually kept constant by simultaneous metered addition of a base and/or acid. The pigments are subsequently separated off, washed and in general dried at 50-150 °C for 6-18 h and optionally calcined for 5-120 minutes, where the calcination temperature can be optimised with respect to the coating present in each case. In general, the calcination temperatures are between 500 and 1000 °C, preferably between 600 and 900 °C. If desired, the pigments can be separated off, dried and optionally calcined after application of the barium sulfate coating and then resuspended for precipitation of the further metal oxide layer.

For preparing the barium sulfate layer a solution containing barium ions, all water-soluble barium salts, such as, for example, barium chloride, barium hydroxide, barium nitrate, can be used. Because of its advantageous price, ready availability and high purity, barium chloride is preferred. For preparing the solution containing sulfate ions, all soluble sulfates, such as, for example, titanium oxysulfate, sodium sulfate, potassium sulfate, magnesium sulfate, sodium bisulfate or potassium bisulfate, as well as sulfuric acid can be used.

The quantities in which the reactants are employed are not in themselves particularly critical. In particular, the quantity of the barium sulfate precipitated onto the substrate or metal oxide layer can be varied within wide limits, depending on the desired properties with regard to adhesive strength, extension and transparency on the skin.

Preferably, the starting suspensions contain about 5-10 weight percent of the flaky substrate, and the sulfate solution and the barium salt solution are added thereto as aqueous solutions containing about 5-25 percent by weight of the respective salt. Preferably, the barium salt is used in a quantity of 0.8-0.98 Ba²⁺ mol per mol of sulfate. After coating with the barium sulfate, the pigment is usually separated off, washed with water and dried.

In the next step a metal oxide or metal hydroxide is also precipitated on, in addition to the barium sulfate layer. The metal-oxide layers are preferably applied by wet-chemical methods, which have been developed for the preparation of pearlescent pigments. Methods of this type are described, for example, in DE 14 67 468, DE 19 59 988, DE 20 09 566, DE 22 14 545, DE 22 15 191, DE 22 44 298, DE 23 13 331, DE 15 22 572, DE 31 37 808, DE 31 37 809, DE 31 51 343, DE 31 51 354, DE 31 51 355, DE 32 11 602, DE 32 35 017 and in further patent documents and other publications known to the person skilled in the art.

As an alternative to the step-wise preparation described above the filler powder can also be prepared by simultaneous or subsequent precipitation of the barium sulfate and the metal salts in a one-pot-process.

If desired, the pigments formed can also be colored with colorants, and they can be very advantageously used together with the additives, conventional for this purpose, in face powders and similar preparations.

By using the foregoing procedures or equivalent ones, the desired firmly adhering coating will be achieved, i.e., the coating will remain on the substrate throughout the conventional use lifetime of the pigments. By small amounts of colored metal oxides a shade of color can be imparted to the pigments, which might be attractive for special uses.

In order to increase the light, water and weather stability, it is frequently advisable to subject the finished filler pigment, depending on the area of use, to post-coating or post-treatment. Suitable post-coatings or posttreatments are, for example, the processes described in German Patent 22 15 191, DE-A 31 51 354, DE-A 32 35 017 or DE-A 33 34 598. This post-coating further increases the chemical and photochemical stability or simplifies handling of the pigment, in particular incorporation into various media. In order to improve the wetability, dispersibility and/or compatibility with the user media, it is possible to apply, for example, functional coatings of Al₂O₃ or ZrO₂ or mixtures thereof to the pigment surface. Also possible are organic post-coatings, for example with silanes, as described, for example, in EP 0 090259, EP 0 634 459, WO 99/57204, WO 96/32446, WO 99/57204, US 5,759,255, US 5,571,851, WO 01/92425 or in J.J. Ponjeé, Philips Technical Review, Vol. 44, No. 3, 81 ff. and P.H. Harding J.C. Berg, J. Adhesion Sci. Technol. Vol. 11 No. 4, pp. 471-493.

The filler pigments according to the invention are versatile and can be employed in many areas. Accordingly, the present invention likewise relates to the use of the filler pigments according to the invention in cosmetics, paints, inks, printing inks, coatings, plastics, films, for the preparation of free-flowing pigment preparations and dry preparations like granules, pellets, etc.

In the case of cosmetics, the filler pigments according to the invention are particularly suitable for products and formulations in skin care products and anti-wrinkle products like make-ups, powders, loose powders, etc.

The concentration of the filler pigments in the application system to be pigmented is generally from 0.5 to 95 % by weight, preferably 1 to 80 % by weight and in particular 5 to 70 % by weight. It is generally dependent on the specific application and can be up to 90 % the case of loose powders. No limits are set for the concentrations of the filler pigments according to the invention in the formulation.

Preferably,
- emulsions contain 0.1-30 % by weight, in particular 1-15 % by weight,
- pigment-containing emulsions comprise 0.1-50% by weight, in particular 1-15 % by weight, depending on the texture,
- toothpastes contain 0.1-60 % by weight, in particular 1-50 % by weight,
- water-free oil/wax-based products comprise 0.1-75 % by weight, in particular 0.5-65 % by weight,
- powder products contain 0.1-95 % by weight, in particular 1-75 % by weight,
of the filler pigments according to the invention, based on the formulation as a whole.

The filler pigments according to the invention can advantageously be employed in both decorative and care cosmetics.

The filler pigments can furthermore be mixed with commercially available state-of-the-art fillers. Fillers which may be mentioned are, for example, natural and synthetic mica, glass beads or glass powder, nylon powder, polymethylmethacrylate powders, pure or filled melamine resins, talc, glasses, kaolin, oxides or hydroxides of aluminium, magnesium, calcium or zinc, BiOCl, barium sulfate, calcium sulfate, calcium carbonate, magnesium carbonate, carbon, boron nitride and physical or chemical combinations of these substances. There are no restrictions regarding the particle shape of the filler. In accordance with requirements, it can be, for example, flake-form, spherical, needle-shaped, crystalline or amorphous.

The filler pigments according to the invention can of course also be combined in the formulations with cosmetic raw materials and auxiliaries of any type. These include, inter alia, oils, fats, waxes, film formers, surfactants, antioxidants, such as, for example, vitamin C or vitamin E, stabilisers, odour intensifiers, silicone oils, emulsifiers, solvents, such as, for example, ethanol, or ethyl acetate or butyl acetate, preservatives and auxiliaries which generally determine applicational properties, such as, for example, thickeners and rheological additives, such as, for example, bentonites, hectorites, silicon dioxides, Ca silicates, gelatines, high-molecular-weight carbohydrates and/or surface-active auxiliaries, etc.

The formulations comprising the pigment mixtures according to the invention can belong to the lipophilic, hydrophilic or hydrophobic type. In the case of heterogeneous formulations having discrete aqueous and nonaqueous phases, the pigment mixtures according to the invention may in each case be present in only one of the two phases or alternatively distributed over both phases.

The pH of the formulations can be between 1 and 14, preferably between 2 and 11 and particularly preferably between 5 and 8.

The pigments according to the invention may furthermore also be combined with cosmetic active ingredients. Suitable active ingredients are, for example, insect repellents, inorganic UV filters, such as, for example, TiO₂, UV A/BC protective filters (for example OMC, B3 and MBC), also in encapsulated form, anti-ageing active ingredients, vitamins and derivatives thereof (for example vitamin A, C, E, etc.), self-tanning agents (for example DHA, erythrulose, inter alia), and further cosmetic active ingredients, such as, for example, bisabolol, LPO, VTA, ectoine, emblica, allantoin, bioflavonoids and derivatives thereof.

Organic UV filters are generally incorporated into cosmetic formulations in an amount of 0.5 to 10 % by weight, preferably 1 to 8 %, and inorganic filters in an amount of 0.1 to 30 %.

The preparations according to the invention may in addition comprise further conventional skin-protecting or skin-care active ingredients. These may in principle be any active ingredients known to the person skilled in the art.

Particularly preferred active ingredients are pyrimidine carboxylic acids and/or aryl oximes.

Of the cosmetic applications, particular mention should be made of the use of ectoine and ectoine derivatives for the care of aged, dry or irritated skin. Thus, European patent application EP-A-0 671 161 describes, in particular, that ectoine and hydroxyectoine are employed in cosmetic preparations, such as powders, soaps, surfactant-containing cleansing products, lipsticks, rouge, make-up, care creams and sunscreen compositions.

Application forms of the cosmetic formulations which may be mentioned are, for example: solutions, suspensions, emulsions, PIT emulsions, pastes, ointments, gels, creams, lotions, powders, soaps, surfactant-containing cleansing compositions, oils, aerosols and sprays. Examples of other application forms are sticks, shampoos and shower preparations. Any desired customary excipients, auxiliaries and, if desired, further active ingredients may be added to the preparation.

Ointments, pastes, creams and gels may comprise the customary excipients, for example animal and vegetable fats, waxes, paraffins, starch, tragacanth, cellulose derivatives, polyethylene glycols, silicones, bentonites, silica, talc and zinc oxide, or mixtures of these substances. Powders and sprays may comprise the customary excipients, for example lactose, talc, silica, aluminium hydroxide, calcium silicate and polyamide powder, or mixtures of these substances. Sprays may additionally comprise the customary propellants, for example chlorofluorocarbons, propane/butane or dimethyl ether.

Solutions and emulsions may comprise the customary excipients, such as solvents, solubilisers and emulsifiers, for example water, ethanol, isopropanol, ethyl carbonate, ethyl acetate, benzyl alcohol, benzyl benzoate, propylene glycol, 1,3-butyl glycol, oils, in particular cottonseed oil, peanut oil, wheatgerm oil, olive oil, castor oil and sesame oil, glycerol fatty acid esters, polyethylene glycols and fatty acid esters of sorbitan, or mixtures of these substances.

Suspensions may comprise the customary excipients, such as liquid diluents, for example water, ethanol or propylene glycol, suspending agents, for example ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Soaps may comprise the customary excipients, such as alkali metal salts of fatty acids, salts of fatty acid monoesters, fatty acid protein hydrolysates, isothionates, lanolin, fatty alcohol, vegetable oils, plant extracts, glycerol, sugars, or mixtures of these substances.

Surfactant-containing cleansing products may comprise the customary excipients, such as salts of fatty alcohol sulfates, fatty alcohol ether sulfates, sulfosuccinic acid monoesters, fatty acid protein hydrolysates, isothionates, imidazolinium derivatives, methyl taurates, sarcosinates, fatty acid amide ether sulfates, alkylamidobetaines, fatty alcohols, fatty acid glycerides, fatty acid diethanolamides, vegetable and synthetic oils, lanolin derivatives, ethoxylated glycerol fatty acid esters, or mixtures of these substances.

Face and body oils may comprise the customary excipients, such as synthetic oils, such as, for example, fatty acid esters, fatty alcohols, silicone oils, natural oils, such as vegetable oils and oily plant extracts, paraffin oils, lanolin oils, or mixtures of these substances.

The cosmetic preparations may exist in various forms. Thus, they can be, for example, a solution, a water-free preparation, an emulsion or microemulsion of the water-in-oil (W/O) or oil-in-water (O/W) type, a multiple emulsion, for example of the water-in-oil-in-water (W/O/W) type, a gel, a solid stick, an ointment or an aerosol. It is also advantageous to administer ectoines in encapsulated form, for example in collagen matrices and other conventional encapsulation materials, for example as cellulose encapsulations, in gelatine, wax matrices or liposomally encapsulated. In particular, wax matrices, as described in DE-A 43 08 282, have proven favourable. Preference is given to emulsions. O/W emulsions are particularly preferred. Emulsions, W/O emulsions and O/W emulsions are obtainable in a conventional manner.

Further embodiments are oily lotions based on natural or synthetic oils and waxes, lanolin, fatty acid esters, in particular triglycerides of fatty acids, or oily-alcoholic lotions based on a lower alcohol, such as ethanol, or a glycerol, such as propylene glycol, and/or a polyol, such as glycerol, and oils, waxes and fatty acid esters, such as triglycerides of fatty acids.

Solid sticks consist of natural or synthetic waxes and oils, fatty alcohols, fatty acids, fatty acid esters, lanolin and other fatty substances.

If a preparation is formulated as an aerosol, the customary propellants, such as alkanes, fluoroalkanes and chlorofluoroalkanes, are generally used.

Cosmetic formulations having light-protection properties may comprise adjuvants, such as surfactants, thickeners, polymers, softeners, preservatives, foam stabilisers, electrolytes, organic solvents, silicone derivatives, oils, waxes, antigrease agents, dyes and/or pigments which colour the composition itself or the hair, or other ingredients usually used in the cosmetic field.

The invention thus furthermore also relates to formulations comprising the filler pigment according to the invention in combination with at least one constituent selected from the group of absorbents, astringents, antimicrobial substances, antioxidants, antiperspirants, antifoaming agents, antidandruff active ingredients, antistatics, binders, biological additives, bleaching agents, chelating agents, deodorants, emollients, emulsifiers, emulsion stabilisers, dyes, humectants, film formers, fillers, odour substances, flavour substances, insect repellents, preservatives, anticorrosion agents, cosmetic oils, solvents, oxidants, vegetable constituents, buffer substances, reducing agents, surfactants, propellant gases, opacifiers, UV filters and UV absorbers, denaturing agents, viscosity regulators, perfume and vitamins

In the case of the use of the filler pigments in paints and coatings, all areas of application known to the person skilled in the art are possible, such as, for example, powder coatings, printing inks for gravure, offset, screen or flexographic printing, toners and for coatings in outdoor applications. The paints and coatings here can be, for example, radiation-curing, physically drying or chemically curing. A multiplicity of binders is suitable for the preparation of printing inks or liquid surface coatings, for example based on acreages, methacrylates, polyesters, polyurethanes, nitrocellulose, ethylcellulose, polyamide, polyvinyl butyrate, phenolic resins, maleic resins, starch or polyvinyl alcohol, amino resins, alkyd resins, epoxy resins, polytetrafluoroethylene, polyvinylidene fluorides, polyvinyl chloride or mixtures thereof, in particular water-soluble grades. The surface coatings can be powder coatings or water- or solvent-based coatings, where the choice of the coating constituents is part of the general knowledge of the person skilled in the art. Common polymeric binders for powder coatings are, for example, polyesters, epoxides, polyurethanes, acrylates or mixtures thereof.

In addition, the filler pigments according to the invention can be used in films and plastics, gift foils, plastic containers and mouldings for all applications known to the person skilled in the art. Suitable plastics for the incorporation of the filler pigments according to the invention are all common plastics, for example thermosets or thermoplastics. The description of the possible applications and the plastics which can be employed, processing methods and additives are given, for example, in RD 472005 or in R. Glausch, M. Kieser, R. Maisch, G. Pfaff, J. Weitzel, Perlglanzpigmente [Pearlescent Pigments], Curt R. Vincentz Verlag, 1996, 83 ff., the disclosure content of which is also incorporated herein.

The filler pigments according to the invention are likewise suitable in the above-mentioned areas of application for use in blends with organic dyes and/or pigments, such as, for example, transparent and opaque white, coloured and black pigments, and with flake-form iron oxides, BiOCl, organic pigments, holographic pigments, LCPs (liquid crystal polymers) and conventional transparent, coloured and black lustre pigments based on metal oxide-coated flakes based on mica, glass, Al₂O₃, Fe₂O₃, SiO₂, metal flakes, etc. The filler pigments according to the invention can be mixed in any ratio with commercially available pigments and fillers.

The filler pigments according to the invention are furthermore suitable for the preparation of flowable pigment compositions and dry preparations comprising one or more particles according to the invention, binders and optionally one or more additives. Dry preparations is also taken to mean preparations which comprise from 0 to 8 % by weight, preferably from 2 to 8 % by weight, in particular from 3 to 6 % by weight, of water and/or a solvent or solvent mixture. The dry preparations are preferably in the form of pellets, granules, chips, sausages or briquettes and have particle sizes of 0.2-80 mm. The dry preparations are used, in particular, in the preparation of printing inks and in cosmetic formulations.

Without further elaboration, it is believed that one skilled in the art can, using the preceding description, utilize the present invention to its fullest extent.

The following preferred specific embodiments are, therefore, to be construed as merely illustrative, and not limitative of the remainder of the disclosure in any way whatsoever.

In the following examples, all temperatures are set forth unconnected in degrees Celsius; unless otherwise indicated, all parts and percentages are by weight.

### Examples

Example: Mica + BaSO₄ + SnO₂ + TiO₂

41,9 g BaCl₂ are dissolved in a suspension of 100 g muscovite mica (<15 µm) in 1400 g of deionized water and heated up to 75°C while stirring. Subsequently, 280 g of a sodium sulfate solution (10 wt.-% of Na₂SO₄) are added with a dosing rate of 5 ml/minute.

After adjusting the pH to 1.8 by addition of hydrochloric acid (10 wt.-% of HCl) a solution of 11.7 g hydrochloric acid (37 wt.-% of HCl) and 18 g tin chloride solution (50 wt.-% of SnCl₄) in 296 g deionized water is added during 60 minutes. Subsequently, 371 g of titanium tetrachloride solution (32 wt.-% TiCl₄) are added with a dosing rate of 1.5 ml/min. During the addition of the tin chloride and titanium chloride solutions the pH is maintained by simultaneous addition of sodium hydroxide solution (32 wt.-% NaOH). After the addition of the titanium chloride solution the pH is elevated to 5.0 with sodium hydroxide solution and stirred for more 15 min.

For work up the product is isolated by filtration and washing with 10 I of deionized water and drying at 110 °C for 12 hours. Finally the product is calcined at 850 °C and sieved through a 40 µm sieve.

The obtained cosmetic filler exhibits bright powder color and an excellent skin feeling.

### Use Examples

### Use Example: Face Powder

| **Ingredients** | | **INCI** | **%** |
|---|---|---|---|
| | | | |
| Phase A | | | |
| Silk Mica | (1) | MICA | 8.00 |
| Filler pigment according | (1) | | 8.00 |
| to Example 1 | | | |
| Ronasphere^{®} LDP | (1) | SILICA, Cl 77891 (TITANIUM DIOXIDE), Cl 77491 (IRON OXIDES) | 5.00 |
| Eusolex^{®} T-S | (1) | TITANIUM DIOXIDE, ALUMINA, STEARIC ACID | 5.00 |
| Unipure Yellow LC 182 | (2) | Cl 77492 (IRON OXIDES) | 1.20 |
| Unipure Red LC 381 | (2 | Cl 77491 (IRON OXIDES) | 0.20 |
| Unipure Brown LC 889 | (2) | Cl 77491 (IRON OXIDES), Cl 77499 (IRON OXIDES) | 0.30 |
| Magnesium stearate | (1) | MAGNESIUM STEARATE | 2.00 |
| Talc | (1) | TALC | 65.90 |
| | | | |
| Phase B | | | |
| RonaCare^{®} Tocopherol Acetate | (1) | TOCOPHERYL ACETATE | 0.30 |
| Fragrance 200 529 | (3) | PARFUM | 0.30 |
| Eutanol G | (4) | OCTYLDODECANOL | 3.70 |
| Propyl-4-hydroxybenzoate | (1) | PROPYLPARABEN | 0.10 |

### Procedure:

Grind the ingredients of phase A until the blend is uniform. Then add the pre-dissolved phase B and grind again until the whole phase A/B is uniform. Fill the bulk into pans and press with the desired pressure. The pressure for pans with 36 mm diameter is approx. 25 bar.

The obtained face powder is a light and transparent formula. Silk Mica delivers more smoothness to the texture while the filler pigment according to Example 1 and the Ronasphere^{®} LDP are responsible for a subtle mattifying and light reflecting effect.

### Suppliers:

(1) Merck KGaA/Rona^{®}
(2) Les Colorants Wackherr
(3) Fragrance Resources
(4) Cognis GmbH

## Claims

1. Filler pigments **characterized in that** a platelet-shaped substrate is coated with barium sulfate and at least two metal oxides and/or metal hydroxides.

2. Filler pigments according to Claim 1 **characterized in that** the substrate is natural or synthetic mica, doped or undoped Al₂O₃ flakes, doped or undoped SiO₂ flakes, talc, kaolin, or doped or undoped glass flakes or mixtures thereof.

3. Filler pigments according to Claim 1 or 2 **characterized in that** the metal oxides and/or metal hydroxides are selected from TiO₂, SnO₂, ZnO, Fe₂O₃, Fe₃O₄, SiO₂, Al2O₃ and ZrO₂.

4. Filler pigments according to at least one of Claims 1 to 3 **characterized in that** the substrates have an aspect ratio of 2 to 2000.

5. Filler pigments according to at least one of Claims 1 to 4 **characterized in that** the amount of barium sulfate is 5-200 wt.-% based on the substrate.

6. Filler pigments according to at least one of Claims 1 to 5 **characterized in that** the substrates have the following layer structure:
| | | |
|---|---|---|
| substrate 1^{st} layer: | BaSO₄ | |
| 2^{nd} layer: | metal oxide 1 | |
| 3^{rd} layer: | metal oxide 2 | or |
| | | |
| substrate 1^{st} layer: | metal oxide 1 | |
| 2^{nd} layer: | BaSO₄ | |
| 3^{rd} layer: | metal oxide 2 | or |
| | | |
| substrate 1^{st} layer: | metal oxide 1 | |
| 2^{nd} layer: | metal oxide 2 | |
| 3^{rd} layer: | BaSO₄ | or |
| | | |
| substrate | | |
| 1^{st} layer: | mixture of BaSO₄ + metal oxide 1 | |
| 2^{nd} layer : | metal oxide 2 | or |
| | | |
| substrate | | |
| 1^{st} layer: | metal oxide 1 | |
| 2^{nd} layer: | mixture of BaSO₄ + metal oxide 2 | or |
| | | |
| substrate | | |
| layer: | mixture of BaSO₄, metal oxide 1 and metal oxide 2. | |

7. Filler pigments according to at least one of Claims 1 to 6 **characterized in that** the surface of the filler pigments is coated with a layer of SiO₂.

8. Filler pigments according to at least one of Claims 1 to 7 **characterized in that** the filler pigments have the following coatings on the surface of the platelet-shaped substrate:
substrate + SnO₂ + TiO₂ + BaSO₄
substrate + mixture of SnO₂ + TiO₂ + BaSO₄
substrate + mixture of SnO₂ + TiO₂ + BaSO₄ + layer of SiO₂ on top
substrate + SiO₂ + TiO₂ + BaSO₄
substrate + SiO₂ + SnO₂ + BaSO₄
substrate + SnO₂ + TiO₂ + BaSO₄ + Carmine red
substrate + SnO₂ + TiO₂ + BaSO₄ + Prussian Blue
substrate + SnO₂ + BaSO₄ / TiO₂
substrate + SnO₂ + TiO₂ + BaSO₄ + SiO₂
substrate + BaSO₄ + SnO₂ + TiO₂ + SiO₂
substrate + Al₂O₃ + BaSO₄ + SnO₂ + SiO₂
substrate + ZnO + Al₂O₃ + BaSO₄ + SiO₂
substrate + Al₂O₃ + BaSO₄ + SnO₂
substrate + ZnO + Al₂O₃ + BaSO₄

9. Process for the production of the filler pigments of at least one of Claims 1 to 8, **characterized in that** the substrate is suspended in an aqueous solution, the barium salt and at least two metal salt solutions are added at a pH which is suitable for hydrolysis and which is selected in such a way that the barium sulfate and the metal oxides and/or hydroxides are precipitated directly onto the substrate.

10. Use of the filler pigments of at least one of Claims 1 to 8 in paints, lacquers, inks, printing inks, plastics and cosmetic formulations.

11. Cosmetic formulations containing up to 95 % by weight of the filler pigments of at least one of Claims 1 to 8.

12. Cosmetic formulations according to Claim 11, **characterised in that**, besides the filler pigments, they comprise at least one constituent selected from the group of the absorbents, astringents, antimicrobial substances, antioxidants, antiperspirants, antifoaming agents, antidandruff active ingredients, antistatics, binders, biological additives, bleaching agents, chelating agents, deodorants, emollients, emulsifiers, emulsion stabilisers, dyes, humectants, film formers, fillers, odour substances, flavour substances, insect repellents, preservatives, anticorrosion agents, cosmetic oils, solvents, oxidants, vegetable constituents, buffer substances, reducing agents, surfactants, propellant gases, opacifiers, UV filters and UV absorbers, denaturing agents, viscosity regulators, perfume and vitamins.

## Patentansprüche

1. Füllpigmente, **dadurch gekennzeichnet, dass** ein plättchenförmiges Substrat mit Bariumsulfat und mindestens zwei Metalloxiden und/oder -hydroxiden beschichtet ist.

2. Füllpigmente nach Anspruch 1, **dadurch gekennzeichnet, dass** es sich bei dem Substrat um natürlichen oder synthetisch hergestellten Glimmer, dotierte oder nicht dotierte Al₂O₃-Plättchen, dotierte oder nicht dotierte SiO₂-Plättchen, Talk, Kaolin oder dotierte oder nicht dotierte Glasplättchen oder Mischungen davon handelt.

3. Füllpigmente nach Anspruch 1 oder 2, **dadurch gekennzeichnet, dass** die Metalloxide und/oder -hydroxide aus TiO₂, SnO₂, ZnO, Fe₂O₃, Fe₃O₄, SiO₂, Al₂O₃ und ZrO₂ ausgewählt sind.

4. Füllpigmente nach mindestens einem der Ansprüche 1 bis 3, **dadurch gekennzeichnet, dass** die Substrate ein Aspektverhältnis von 2 bis 2000 aufweisen.

5. Füllpigmente nach mindestens einem der Ansprüche 1 bis 4, **dadurch gekennzeichnet, dass** die Menge an Bariumsulfat 5-200 Gew.-%, bezogen auf das Substrat, beträgt.

6. Füllpigmente nach mindestens einem der Ansprüche 1 bis 5, **dadurch gekennzeichnet, dass** die Substrate die folgende Schichtstruktur aufweisen:
| | | |
|---|---|---|
| Substrat 1. Schicht: | _{B}aSO₄ | |
| 2. Schicht: | Metalloxid 1 | |
| 3. Schicht: | Metalloxid 2 | oder |
| Substrat | | |
| 1. Schicht: | Metalloxid 1 | |
| 2. Schicht: | BaSO₄ | |
| 3. Schicht: | Metalloxid 2 | oder |
| | | |
| Substrat | | |
| 1. Schicht: | Metalloxid 1 | |
| 2. Schicht: | Metalloxid 2 | |
| 3. Schicht: | BaSO₄ | oder |
| | | |
| Substrat | | |
| 1. Schicht: | Mischung aus BaSO₄ + Metalloxid 1 | |
| 2. Schicht: | Metalloxid 2 | oder |
| | | |
| Substrat | | |
| 1. Schicht: | Metalloxid 1 | |
| 2. Schicht: | Mischung aus BaSO₄ + Metalloxid 2 | oder |
| | | |
| Substrat | | |
| Schicht: | Mischung aus BaSO₄, Metalloxid 1 und Metalloxid 2. | |

7. Füllpigmente nach mindestens einem der Ansprüche 1 bis 6, **dadurch gekennzeichnet, dass** die Oberfläche der Füllpigmente mit einer Schicht aus SiO₂ beschichtet ist.

8. Füllpigmente nach mindestens einem der Ansprüche 1 bis 7, **dadurch gekennzeichnet, dass** die Füllpigmente die folgenden Beschichtungen auf der Oberfläche des plättchenförmigen Substrates aufweisen:
Substrat + SnO₂ + TiO₂ + BaSO₄
Substrat + Mischung aus SnO₂ + TiO₂ + BaSO₄
Substrat + Mischung aus SnO₂ + TiO₂ + BaSO₄ + Deckschicht aus SiO₂
Substrat + SiO₂ + TiO₂ + BaSO₄
Substrat + SiO₂ + SnO₂ + BaSO₄
Substrat + SnO₂ + TiO₂ + BaSO₄ + Karminrot
Substrat + SnO₂ + TiO₂ + BaSO₄ + Berliner Blau
Substrat + SnO₂ + BaSO₄ / TiO₂
Substrat + SnO₂ + TiO₂ + BaSO₄ + SiO₂
Substrat + BaSO₄ + SnO₂ + TiO₂ + SiO₂
Substrat + Al₂O₃ +BaSO₄ + SnO₂ + SiO₂
Substrat + ZnO + Al₂O₃ +BaSO₄ + SiO₂
Substrat + Al₂O₃ +BaSO₄ + SnO₂
Substrat + ZnO + Al₂O₃ +BaSO₄

9. Verfahren zur Herstellung der Füllpigmente nach mindestens einem der Ansprüche 1 bis 8, **dadurch gekennzeichnet, dass** das Substrat in einer wässrigen Lösung suspendiert wird, das Bariumsalz und mindestens zwei Metallsalzlösungen bei einem pH-Wert zugegeben werden, der für die Hydrolyse geeignet und so ausgewählt ist, dass das Bariumsulfat und die Metalloxide und/oder -hydroxide direkt auf das Substrat gefällt werden.

10. Verwendung der Füllpigmente nach mindestens einem der Ansprüche 1 bis 8 in Farben, Lacken, Tinten, Druckfarben, Kunststoffen und Kosmetikformulierungen.

11. Kosmetikformulierungen, die bis zu 95 Gew.-% der Füllpigmente nach mindestens einem der Ansprüche 1 bis 8 enthalten.

12. Kosmetikformulierungen nach Anspruch 11, **dadurch gekennzeichnet, dass** sie neben den Füllpigmenten mindestens einen Bestandteil ausgewählt aus der Gruppe der Absorbentien, Adstringentien, antimikrobiellen Substanzen, Antioxidantien, Antiperspirantien, Antischaummittel, Antischuppenwirkstoffe, Antistatika, Bindemittel, biologischen Zusätze, Bleichmittel, Chelatbildner, Deodorantien, Emollentien, Emulgatoren, Emulsionsstabilisatoren, Farbstoffe, Feuchthaltemittel, Filmbildner, Füllstoffe, Geruchsstoffe, Geschmacksstoffe, Insektenschutzmittel, Konservierungsstoffe, Korrosionsschutzmittel, kosmetischen Öle, Lösungsmittel, Oxidantien, pflanzlichen Bestandteile, Puffersubstanzen, Reduktionsmittel, Tenside, Treibgase, Trübungsmittel, UV-Filter und -Absorber, Denaturierungsmittel, Viskositätsregler, Parfüme und Vitamine enthalten.

## Revendications

1. Pigments de charge, **caractérisé en ce qu'**un substrat sous forme de feuillets est revêtu de sulfate de baryum et d'au moins deux oxydes métalliques et/ou hydroxydes métalliques.

2. Pigments de charge selon la revendication 1, **caractérisés en ce que** le substrat est du mica naturel ou synthétique, des paillettes de Al₂O₃ dopé ou non dopé, des paillettes de SiO₂ dopé ou non dopé, du talc, du kaolin ou des paillettes de verre dopé ou non dopé, ou des mélanges de ceux-ci.

3. Pigments de charge selon la revendication 1 ou 2, **caractérisés en ce que** les oxydes métalliques et/ou les hydroxydes métalliques sont sélectionnés parmi TiO₂, SnO₂, ZnO, Fe₂O₃, Fe₃O₄, SiO₂, Al₂O₃ et ZrO₂.

4. Pigments de charge selon au moins l'une des revendications 1 à 3, **caractérisés en ce que** les substrats ont un rapport d'aspect allant de 2 à 2000.

5. Pigments de charge selon au moins l'une des revendications 1 à 4, **caractérisés en ce que** la quantité de sulfate de baryum est de 5-200% en poids du poids du substrat.

6. Pigments de charge selon au moins l'une des revendications 1 à 5, **caractérisés en ce que** les substrats ont la structure de couches suivante :
| | | |
|---|---|---|
| substrat | | |
| 1^{ère} couche : | BaSO₄ | |
| 2^{ème} couche : | oxyde métallique 1 | |
| 3^{ème} couche : | oxyde métallique 2 | ou |
| | | |
| substrat | | |
| 1^{ère} couche : | oxyde métallique 1 | |
| 2^{ème} couche: | BaSO₄ | |
| 3^{ème} couche : | oxyde métallique 2 | ou |
| | | |
| substrat | | |
| 1^{ère} couche : | oxyde métallique 1 | |
| 2^{ème} couche : | oxyde métallique 2 | |
| 3^{ème} couche : | BaSO₄ | ou |
| | | |
| substrat | | |
| 1^{ère} couche : | mélange de BaSO₄ + oxyde métallique 1 | |
| 2^{ème} couche : | oxyde métallique 2 | ou |
| | | |
| substrat | | |
| 1^{ère} couche : | oxyde métallique 1 | |
| 2^{ème} couche : | mélange de BaSO₄ + oxyde métallique 2 | ou |
| | | |
| substrat | | |
| couche : | mélange de BaSO₄, d'oxyde métallique 1 et d'oxyde métallique 2. | |

7. Pigments de charge selon au moins l'une des revendications 1 à 6, **caractérisés en ce que** la surface des pigments de charge est revêtue d'une couche de SiO₂.

8. Pigments de charge selon au moins l'une des revendications 1 à 7, **caractérisés en ce que** les pigments de charge comportent les revêtements suivants à la surface du substrat sous forme de feuillets :
substrat + SnO₂ + TiO₂ + BaSO₄
substrat + mélange de SnO₂ + TiO₂ + BaSO₄
substrat + mélange de SnO₂ + TiO₂ + BaSO₄ + couche de SiO₂ au dessus
substrat + SiO₂ + TiO₂ + BaSO₄
substrat + SiO₂ + SnO₂ + BaSO₄
substrat + SnO₂ + TiO₂ + BaSO₄ + rouge carmin
substrat + SnO₂ + TiO₂ + BaSO₄ + bleu de Prusse
substrat + SnO₂ + BaSO₄/TiO₂
substrat + SnO₂ + TiO₂ + BaSO₄ + SiO₂
substrat + BaSO₄ + SnO₂ + TiO₂ + SiO₂
substrat + Al₂O₃ + BaSO₄ + SnO₂ + SiO₂
substrat + ZnO + Al₂O₃ + BaSO₄ + SiO₂
substrat + Al₂O₃ + BaSO₄ + SnO₂
substrat + ZnO + Al₂O₃ + BaSO₄.

9. Procédé de production des pigments de charge selon au moins l'une des revendications 1 à 8, **caractérisé en ce que** le substrat est mis en suspension dans une solution aqueuse, le sel de baryum et au moins deux solutions de sels métalliques sont ajoutés à un pH convenable pour l'hydrolyse et qui est sélectionné de manière telle que le sulfate de baryum et les oxydes et/ou hydroxydes métalliques soient précipités directement sur le substrat.

10. Utilisation des pigments de charge selon au moins l'une des revendications 1 à 8 dans les peintures, les laques, les encres, les encres d'impression, les matières plastiques et les formulations cosmétiques.

11. Formulations cosmétiques contenant jusqu'à 95% en poids des pigments de charge selon au moins l'une des revendications 1 à 8.

12. Formulations cosmétiques selon la revendication 11, **caractérisées en ce que**, outre les pigments de charge, elles comprennent au moins un constituant sélectionné dans le groupe constitué par les absorbants, les astringents, les substances antimicrobiennes, les antioxydants, les agents anti-transpirants, les agents anti-mousse, les ingrédients actifs antipelliculaires, les agents antistatiques, les liants, les additifs biologiques, les agents de blanchiment, les agents chélatants, les déodorants, les émollients, les émulsifiants, les stabilisants d'émulsions, les colorants, les humectants, les agents filmogènes, les charges, les substances odorantes, les agents aromatisants, les agents répulsifs contre les insectes, les agents conservateurs, les agents anticorrosifs, les huiles cosmétiques, les solvants, les agents oxydants, les constituants végétaux, les substances tampon, les agents réducteurs, les tensioactifs, les gaz propulseurs, les agents opacifiants, les filtres UV et les absorbants UV, les agents dénaturants, les régulateurs de viscosité, les parfums et les vitamines.
